# EUROPÄISCHE PATENTSCHRIFT

(11) **EP 2 950 846 B1**
(45) Veröffentlichungstag und Bekanntmachung des Hinweises auf die Patenterteilung: **25.10.2017**
(21) Anmeldenummer: 14700742.1
(22) Anmeldetag: 17.01.2014
(51) Int. Cl.: A61M 1/30

(54) **EXTRAKORPORALE BLUTBEHANDLUNGSVORRICHTUNG FÜR DEN BETRIEB MIT EINEM EINZIGEN PATIENTENANSCHLUSS UND VERFAHREN ZUM BETREIBEN EINER EXTRAKORPORALEN BLUTBEHANDLUNGSVORRICHTUNG MIT EINEM EINZIGEN PATIENTENANSCHLUSS**
EXTRACORPOREAL BLOOD TREATMENT DEVICE FOR THE OPERATION HAVING A SINGLE PATIENT CONNECTION AND METHOD FOR OPERATING AN EXTRACORPOREAL BLOOD TREATMENT DEVICE HAVING A SINGLE PATIENT CONNECTION
DISPOSITIF EXTRACORPOREL DE TRAITEMENT DU SANG FONCTIONNANT AVEC UN SEUL RACCORDEMENT AU PATIENT, ET PROCÉDÉ PERMETTANT DE FAIRE FONCTIONNER UN DISPOSITIF EXTRACORPOREL DE TRAITEMENT DU SANG FONCTIONNANT AVEC UN SEUL RACCORDEMENT AU PATIENT

(30) Priorität: 29.01.2013 DE 102013001437; 29.01.2013 US 201361757773 P
(43) Veröffentlichungstag der Anmeldung: 09.12.2015
(73) Patentinhaber: Fresenius Medical Care Deutschland GmbH, 61352 Bad Homburg (DE)
(72) Erfinder: BOCKLET, Christoph, 97708 Bad Bocklet (DE)
(74) Vertreter: Oppermann, Frank
(86) Internationale Anmeldenummer: PCT/EP2014/050928
(87) Internationale Veröffentlichungsnummer: WO 2014/118021

(56) Entgegenhaltungen:
- EP-A1- 0 498 741
- EP-A1- 2 415 491
- EP-A1- 2 465 553
- WO-A1-2010/037520
- WO-A2-2008/148506
- US-A- 4 231 366

## Beschreibung

Die Erfindung betrifft eine extrakorporale Blutbehandlungsvorrichtung für den Betrieb mit einem einzigen Patientenanschluss, der mit einer arteriellen und einer venösen Blutleitung eines extrakorporalen Blutkreislaufs verbunden ist.

Blutbehandlungsvorrichtungen mit einer Blutbehandlungseinheit, die von dem Blut eines Patienten durchströmt wird, sind allgemein bekannt. Zu diesen zählen beispielsweise die bekannten Hämodialyse-, Hämofiltrations- oder Hämodiafiltrationsvorrichtungen. Blutbehandlungsvorrichtungen können mit getrennten arteriellen und venösen Patientenanschlüssen oder mit einem einzigen Patientenanschluss betrieben werden.

Bei dem Betrieb einer Blutbehandlungsvorrichtung mit einem einzigen Patientenanschluss erfolgt die Entnahme und Rückführung des Bluts über eine einzige Kanüle, an der sowohl die arterielle als auch die venöse Blutleitung angeschlossen sind. Das dem Patienten entnommene Blut wird während einer arteriellen Phase in einem Reservoir gespeichert und in einer venösen Phase aus dem Speicher dem Patienten wieder zugeführt.

Aus der WO 2008/148506 A2 ist eine extrakorporale Blutbehandlungsvorrichtung bekannt, bei der in der arteriellen Phase Blut in die Blutbehandlungseinheit und aus der Blutbehandlungseinheit in einen Blutsammelbehälter gefördert wird, wobei die Blutzufuhr zum Patienten unterbrochen ist. In dem Blutsammelbehälter wird ein vorgegebener Überdruck aufgebaut, der überwacht wird. Aufgrund des Überdrucks wird das in dem Blutsammelbehälter gesammelte Blut in der venösen Phase aus dem Blutsammelbehälter verdrängt und dem Patienten zugeführt, während die Blutzufuhr zu der Blutbehandlungseinheit unterbrochen ist, so dass die Blutbehandlungseinheit während der venösen Phase nicht von Blut durchströmt wird.

Wenn der Dialysator einer Dialysevorrichtung nicht kontinuierlich von Blut durchströmt wird, besteht die Gefahr, dass die Membran des Dialysators verstopft (clotting). Es sind Blutbehandlungsvorrichtungen für den Einnadel-Betrieb bekannt, bei denen eine kontinuierliche Durchströmung des Dialysators mit Blut erfolgt.

Das Problem der Verstopfung der Dialysatormembran bei der Einnadel-Dialyse wird in der DE 42 17 692 A1 angesprochen, wobei eine Ventilanordnung zur Steuerung der Flüssigkeitsströmung vorgeschlagen wird. In einem vorausgehenden Schritt wird Blut vom Patienten mit einer Blutpumpe über den Dialysator in einen Pufferbehälter gepumpt und in einem nachfolgenden Schritt in der gleichen Flussrichtung aus dem Pufferbehälter über den Dialysator wieder zum Patienten gepumpt. Zwischen beiden Schritten zirkuliert das Blut über den Dialysator in einem geschlossenen Kreislauf, von dem der Patient aber mit der Ventilanordnung abgetrennt wird, so dass die Blutströmung durch den Dialysator nicht unterbrochen ist.

Aus der WO 2010/037520 A1 ist eine extrakorporale Blutbehandlungsvorrichtung für den Einnadel-Betrieb mit einem extrakorporalen Blutkreislauf bekannt, der über zwei okkludierende Pumpen verfügt, die in der arteriellen Blutleitung stromauf und stromab eines Blutspeichers angeordnet sind. Beide Blutpumpen werden in der arteriellen und venösen Phase mit unterschiedlichen Flussraten betrieben. In der arteriellen Phase wird für die Blutpumpe stromauf des Blutspeichers eine größere Förderrate als für die Blutpumpe stromab des Speichers eingestellt, so dass dem Patienten Blut entnommen wird. In der venösen Phase hingegen ist die Förderrate der Blutpumpe stromauf des Blutspeichers kleiner als die Förderrate der Pumpe stromab des Speichers, so dass dem Patienten Blut zugeführt wird. Die bekannte Vorrichtung ist dazu bestimmt, relativ kleine Blutflüsse einstellen zu können. Nachteilig ist, dass ein Transmembranfluss über die Membran des Dialysators möglich ist.

Die DE 2 236 433 beschreibt eine Dialysevorrichtung für den Einnadel-Betrieb mit einem extrakorporalen Blutkreislauf, bei der die Blutpumpe, die in der arteriellen Blutleitung stromauf des Dialysators angeordnet ist, während der arteriellen und venösen Phase betrieben wird, so dass durch den Dialysator Blut kontinuierlich gefördert wird. Die Dialysevorrichtung sieht einen arteriellen Blutspeicher und eine Druckkammer vor. In der arteriellen Phase ist die arterielle Schlauchklemme in der arteriellen Blutleitung geöffnet, während die venöse Schlauchklemme in der venösen Blutleitung geschlossen ist, so dass Blut vom Patienten in die Druckkammer gepumpt wird. Wenn der Duck in der Kammer einen vorgegebenen Wert erreicht hat, wird in der venösen Phase die venöse Schlauchklemme geöffnet und die arterielle Schlauchklemme geschlossen, so dass das Blut aus der Druckkammer dem Patienten zugeführt wird. Als nachteilig erweisen sich die stromauf des Dialysators auftretenden erheblichen Druckänderungen und Druckspitzen, die zu einem unerwünschten und unkontrollierten Blutplasmaaustausch über die Membran des Dialysators führen können, wodurch die Gefahr des Verstopfens der Dialysatormembran besteht.

Die US 4 231 366 A beschreibt eine extrakorporale Blutbehandlungsvorrichtung für den Einnadel-Betrieb, die über eine arterielle Pumpe in der arteriellen Blutleitung und eine venöse Pumpe in der venösen Blutleitung verfügt. Stromab des Dialysators und stromauf der venösen Pumpe ist in der venösen Blutleitung ein Reservoir angeordnet, aus dem die venöse Pumpe in der venösen Phase das Blut des Patienten fördert. Bei einer alternativen Ausführungsform ist ein Betrieb vorgesehen, bei dem die arterielle Pumpe sowohl in der arteriellen als auch in der venösen Phase und die venöse Pumpe nur in der venösen Phase betrieben wird. Dabei soll die Förderarte der venösen Pumpe größer als die der arteriellen Pumpe sein. Die Blutflussraten beider Pumpe sollen auch durch ein arterielles und venöses Absperrorgan noch beeinflusst werden können.

Aus der EP 2 415 491 A1 ist eine Blutbehandlungsvorrichtung für den Einnadel-Betrieb bekannt, die anstelle einer venösen Pumpe in der venösen Blutleitung über ein Reservoir verfügt, das mit einer Luftpumpe in Fluidverbindung steht. Die Blutbehandlungsvorrichtung verfügt nicht über ein arterielles und venöses Absperrorgan. Das Funktionsprinzip der Blutbehandlungsvorrichtung liegt darin, dass die arterielle Pumpe sowohl in der arteriellen als auch venösen Phase betrieben wird, wobei die Flussrate in der arteriellen Phase größer als in der venösen Phase ist, so dass sich das Reservoir mit Blut füllt. Die Luftpumpe arbeitet in einem alternierenden Betrieb in beide Richtungen, wobei das Reservier in der arteriellen Phase entleert und in der venösen Phase Der Erfindung liegt die Aufgabe zu Grunde, eine extrakorporale Blutbehandlungsvorrichtung zu schaffen, die einen Betrieb mit einem einzigen Patientenanschluss ohne wesentliche Druckänderungen und Druckspitzen erlaubt.

Eine weitere Aufgabe der vorliegenden Erfindung ist es, eine extrakorporale Blutbehandlungsvorrichtung für eine Single-Needle-Behandlung zu schaffen, bei der die Gefahr der Gerinnselbildung des Bluts (clotting) im extrakorporalen Blutkreislauf weiter verringert wird.

Wiederum eine weitere Aufgabe der vorliegenden Erfindung ist es, eine extrakorporale Blutbehandlungsvorrichtung für eine Single-Needle-Behandlung zu schaffen, bei der die erforderliche Menge von Antikoagulanzmittel, beispielsweise von Heparin, verringert ist, Wiederum eine weitere Aufgabe der vorliegenden Erfindung ist es, eine extrakorporale Blutbehandlungsvorrichtung für eine Single-Needle-Behandlung zu schaffen, bei der die Clearance des behandelten Bluts weiter verbessert wird.

Wiederum eine weitere Aufgabe der vorliegenden Erfindung ist es, eine extrakorporale Blutbehandlungsvorrichtung für eine Single-Needle-Behandlung zu schaffen, bei der ein Hämodiafiltrationsbetrieb mit Zugabe von "online" gestellter Substitutionsflüssigkeit vor dem Dialysator und/oder nach dem Dialysator während der arteriellen Phase und während der venösen Phase möglich ist.

Die Lösung dieser Aufgaben erfolgt erfindungsgemäß mit den Merkmalen der unabhängigen Patentansprüche. Die Gegenstände der Unteransprüche betreffen vorteilhafte Ausführungsformen der Erfindung.

Die erfindungsgemäße Blutbehandlungsvorrichtung verfügt über zwei Einrichtungen zum Fördern von Blut in der arteriellen und venösen Blutleitung. Die erste Einrichtung zum Fördern von Blut kann unterschiedlich beschaffen sein, während die zweite Einrichtung zum Fördern von Blut Mittel zum Sammeln von Blut und Mittel zum Aufbau eines Drucks in den Mitteln zum Sammeln von Blut aufweist, so dass in den Mitteln zum Sammeln von Blut gesammeltes Blut zu dem Patientenanschluss strömt. Darüber hinaus verfügt die Blutbehandlungsvorrichtung über ein arterielles und venöses Absperrorgan zum Unterbrechen der Flüssigkeitsströmung in der arteriellen bzw. venösen Blutleitung sowie eine Steuereinheit zum Ansteuern der beiden Einrichtungen zum Fördern von Blut und des arteriellen und venösen Absperrorgans.

Die erfindungsgemäße Blutbehandlungsvorrichtung zeichnet sich dadurch aus, dass die erste Einrichtung zum Fördern von Blut sowohl während der arteriellen als auch der venösen Phase betrieben wird, so dass die Blutbehandlungseinheit kontinuierlich von Blut durchströmt wird. In der arteriellen Phase wird Blut mit einer vorgegebenen ersten Flussrate von dem Patientenanschluss über die Blutbehandlungseinheit zu den Mitteln zum Sammeln von Blut mit der ersten Einrichtung gefördert, wobei die Flüssigkeitsströmung in der venösen Blutleitung zu dem Patientenanschluss unterbrochen wird. An die arterielle Phase schließt sich die venöse Phase an, in der Blut mit einer vorgegebenen zweiten Flussrate über die Blutbehandlungseinheit in die Mittel zum Sammeln von Blut mit der ersten Einrichtung gefördert wird.

In der arteriellen und venösen Phase strömt das Blut über die Blutbehandlungseinheit mit der ersten bzw. zweiten Flussrate, wobei dem Patienten in der arteriellen Phase ein bestimmtes Volumen an Blut entnommen wird. Dieses Volumen an Blut wird dem Patienten in der venösen Phase mit der zweiten Einrichtung zum Fördern von Blut wieder zugeführt. In den Mitteln zum Sammeln von Blut wird hierzu Druck aufgebaut, so dass Blut mit einer vorgegebenen dritten Flussrate, die größer als die erste bzw. zweite Flussrate ist, aus den Mitteln zum Sammeln von Blut zu dem Patientenanschluss strömt. An dem Patientenanschluss teilt sich der Blutfluss in den Zirkulationsfluss und den Rückgabefluss auf. Für die Förderung von Blut ist der Aufbau eines Überdrucks in den Mitteln zum Sammeln von Blut erforderlich. Für die Regelung ist unerheblich, ob mit den Mitteln zum Aufbau eines Drucks ein bestimmter Volumenstrom vorgegeben wird, so dass sich ein korrespondierender Rückgabedruck einstellt, oder ein bestimmter Rückgabedruck eingestellt wird, so dass sich ein korrespondierender Fluss einstellt.Das in der venösen Phase dem Patienten zugeführte Volumen an Blut ist von der Flussrate unabhängig, mit der das Blut durch den geschlossenen Kreislauf strömt, der die arterielle und venöse Blutleitung und die Blutbehandlungseinheit umfasst. Folglich kann die erste Einrichtung zum Fördern von Blut in der arteriellen und venösen Phase mit der gleichen oder mit unterschiedlichen Förderraten betrieben werden. Die dritte Flussrate ergibt sich aus der Summe des Volumenstroms von Blut, das mit der ersten Einrichtung gefördert wird und dem Volumenstrom von Blut, das aus den Mitteln zum Sammeln von Blut aufgrund des Rückgabedrucks verdrängt wird.

Der Druck im extrakorporalen Blutkreislauf, insbesondere in der Blutbehandlungseinheit, kann während der arteriellen und venösen Phase konstant gehalten werden. In der venösen Phase kann sich ein Rückgabedruck bei einem konstanten Blutfluss einstellen, während in der arteriellen Phase dieser Rückgabedruck mit den Mitteln zum Aufbau des Drucks eingestellt werden kann. Hierdurch werden unerwünschte Druckschwankungen und Druckspitzen vermieden. Die Blutflüsse in der venösen und arteriellen Phase können unabhängig voneinander frei eingestellt werden. Weiterhin kann der Zirkulationsfluss unabhängig vom venösen und arteriellen Fluss eingestellt werden.

Die erste und zweite Flussrate, mit der Blut während der arteriellen und venösen Phase von dem Patientenanschluss über die Blutbehandlungseinheit zu den Mitteln zum Sammeln von Blut gefördert wird, können gleich oder unterschiedlich sein. Folglich kann die erste Flussrate größer oder kleiner als die zweite Flussrate sein. In der Praxis werden aber beide Flussraten im Wesentlichen gleich sein. Die Flussrate in der arteriellen Phase kann geringfügig niedriger sein als in der venösen Phase, um ein Ansaugen der arteriellen Nadel an der Wandung der Fistel oder des Shunts des Patienten aufgrund des Unterdrucks auf der Saugseite der Blutpumpe zu vermeiden.

Für die erfindungsgemäße Blutbehandlungsvorrichtung ist entscheidend, dass während der venösen Phase Blut mit einer größeren Flussrate zu dem Patientenanschluss und mit einer kleineren Flussrate von dem Patientenanschluss strömt, so dass dem Patienten Blut zugeführt wird. In der arteriellen Phase strömt Blut nur von dem Patientenanschluss in die Mittel zum Sammeln von Blut, da die Blutzufuhr zu dem Patientenanschluss unterbrochen ist.

Für die Erfindung ist unerheblich, wo die arterielle und venöse Blutleitung zu dem Patientenanschluss zusammengeführt werden. In der Praxis wird die Länge des Leitungsabschnitts zwischen der Zusammenführung von arterieller und venöser Leitung und dem Patienten aber möglichst kurz gehalten, weil das Volumen dieses Leitungsabschnitts nach der venösen Phase mit bereits dialysiertem Blut gefüllt ist, so dass dieses Volumen das gesamte Schlagvolumen nachteilig schmälert und die Effizienz der Behandlung verringert.

Bei einer bevorzugten Ausführungsform der Erfindung ist der einzige Patientenanschluss als eine Kanüle mit einem distalen und proximalen Ende ausgebildet, wobei an dem proximalen Ende der Kanüle ein die arterielle und venöse Blutleitung verbindendes Verbindungstück vorgesehen ist. Es ist aber auch möglich, dass das Verbindungsstück für die Blutleitungen nicht Teil des Patientenanschlusses ist.

Die erste Einrichtung zum Fördern von Blut kann unterschiedlich ausgebildet sein. Vorzugsweise ist die erste Einrichtung zum Fördern von Blut eine okkludierende Blutpumpe.

Eine besonders bevorzugte Ausführungsform der Erfindung sieht vor, dass die zweite Einrichtung zum Fördern von Blut Mittel zum Speichern von Luft aufweist, die ein abgeschlossenes Volumen umfassen, wobei die Mittel zum Aufbau eines Drucks derart mit den Mitteln zum Speichern von Luft und den Mitteln zum Sammeln von Blut in Strömungsverbindung stehen, dass Luft aus den Mitteln zum Speichern von Luft unter Verdrängung des in den Mitteln zum Sammeln von Blut gesammelten Bluts in die Mittel zum Sammeln von Blut überführbar ist. Bei dieser Ausführungsform wird ein abgeschlossenes Volumen geschaffen, das die Mittel zum Speichern von Luft und die Mittel zum Sammeln von Blut zusammen mit den zugehörigen Verbindungsleitungen einschließt. In dieses abgeschlossene Volumen kann grundsätzlich keine Luft eindringen bzw. austreten.

Die Mittel zum Verdichten von Luft können unterschiedlich ausgebildet sein, beispielsweise kann ein Kompressor zum Verdichten von Luft vorgesehen sein.

Eine weitere besonders bevorzugte Ausführungsform sieht vor, dass die zweite Einrichtung zum Fördern von Blut eine die Mittel zum Speichern von Luft und die Mittel zum Sammeln von Blut verbindende Bypassleitung aufweist, wobei in die Bypassleitung ein Bypassventil angeordnet ist. In der arteriellen Phase wird das Bypassventil geöffnet, so dass Luft aus den Mitteln zum Sammeln von Blut in die Mittel zum Speichern von Luft gefördert wird, während in der venösen Phase das Bypassventil geschlossen wird, so dass mit den Mitteln zum Aufbau eines Überdrucks die in den Mitteln zum Speichern von Luft befindliche Luft in die Mittel zum Sammeln von Blut überführt wird, so dass das Blut aus den Mitteln zum Sammeln von Blut verdrängt wird. Das Bypassventil kann in der arteriellen Phase gemäß einer Druckregelung getaktet werden.

Bei der erfindungsgemäßen Blutbehandlungsvorrichtung ist die Steuereinheit derart konfiguriert, dass sämtliche erforderliche Komponenten derart angesteuert werden, dass die einzelnen Verfahrensschritte durchgeführt werden. Die Steuereinheit kann eine separate Einheit oder Bestandteil der zentralen Steuereinheit der extrakorporalen Blutbehandlungsvorrichtung sein. Beispielsweise kann die Steuereinheit einen Mikroprozessor aufweisen, auf dem ein Datenverarbeitungsprogramm (Software) läuft.

Die erfindungsgemäße Vorrichtung erlaubt die Einstellung größerer Blutflüsse über die Blutbehandlungseinheit als der Blutfluss, mit dem Blut dem Patienten zurückgegeben wird, wodurch sich eine verbesserte Clearance ergibt. Es sind weiterhin längere Zykluszeiten möglich, da das Blut nicht zum Stillstand kommt. Aufgrund der geringeren Gefahr des Verstopfens der Membran des Dialysators (clotting), ist die Verabreichung von geringeren Mengen von Heparin möglich.

Bei der erfindungsgemäßen Vorrichtung ist während der arteriellen Phase das venöse Absperrorgan geschlossen. Dadurch wird eine unerwünschte Rezirkulation sicher verhindert.

Im Folgenden wird ein Ausführungsbeispiel der Erfindung unter Bezugnahme auf die Zeichnungen im Einzelnen erläutert.

Es zeigen:
- Fig. 1: Die wesentlichen Komponenten der erfindungsgemäßen extrakorporalen Blutbehandlungsvorrichtung für den Einnadel-Betrieb in stark vereinfachter schematicher Darstellung und

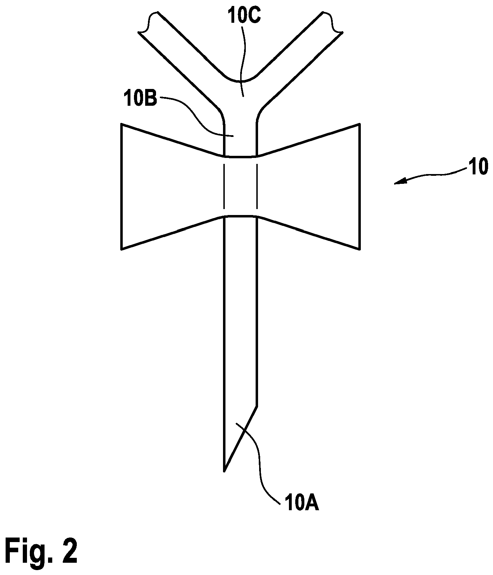

- Fig. 2: einen Ausschnitt von Fig. 1 in vergrößerter Darstellung.

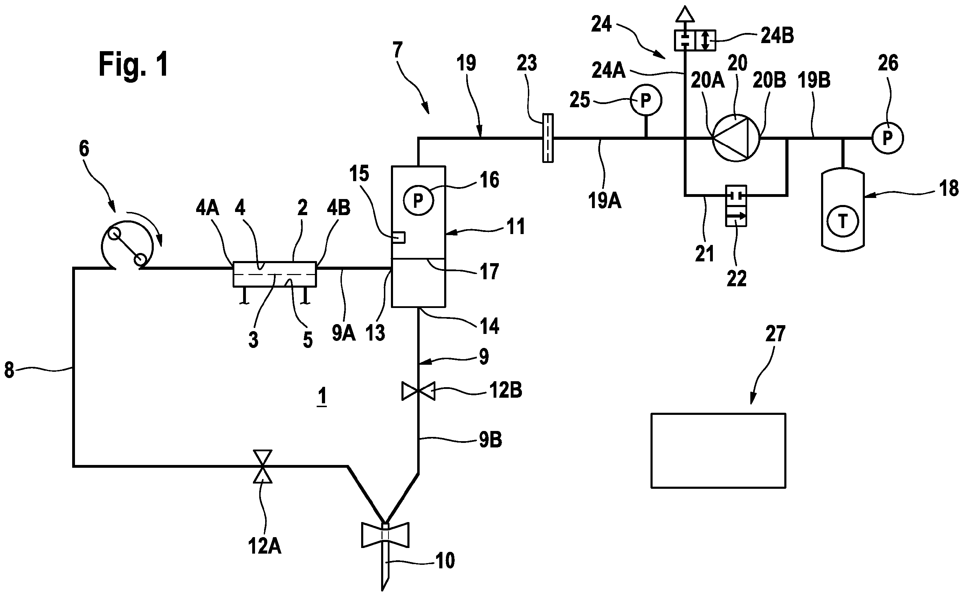

Fig. 1 zeigt die wesentlichen Komponenten der Blutbehandlungsvonichtung für den Einnadel-Betrieb, insbesondere Dialysevorrichtung, in stark vereinfachter schematischer Darstellung. Die einzelnen Komponenten der Blutbehandlungsvorrichtung sind in der WO 2008/148506 A2 im Einzelnen beschrieben, auf die ausdrücklich Bezug genommen wird.

Die Blutbehandlungsvorrichtung verfügt über einen extrakorporalen Blutkreislauf 1 mit einer Blutbehandlungseinheit 2, beispielsweise einen Dialysator. Der Dialysator 2 ist durch eine semipermeable Membran 3 in eine Blutkammer 4 und eine Dialysierflüssigkeitskammer 5 unterteilt. In dem extrakorporalen Blutkreislauf 1 wird das Blut des Patienten mittels einer ersten Einrichtung 6 zum Fördern von Blut und einer zweiten Einrichtung 7 zum Fördern von Blut gefördert. Die erste Einrichtung 6 zum Förden von Blut ist vorzugsweise eine okkludierende Schlauchpumpe. Der Dialysierflüssigkeitskreislauf ist in Fig. 1 nicht dargestellt.

In die Blutbehandlungsvorrichtung wird ein Schlauchset eingelegt, das nach der Behandlung verworfen wird. Das Schlauchset weist eine zu dem Einlass 4A der Blutkammer 4 des Dialysators 2 führende arterielle Blutzuführleitung 8, die in die okkludierende Schlauchpumpe 6 eingelegt ist, und eine von dem Auslass 4B der Blutkammer abgehende venöse Blutrückführleitung 9 auf. Blutzuführleitung und Blutrückführleitung 8, 9 sind an eine gemeinsamen Patientenanschluss 10 angeschlossen. Der Patientenanschluss 10 ist eine Kanüle mit einem distalen und proximalen Ende 10A, 10B. Das proximale Ende 10B der Kanüle 10 ist als ein Verbindungsstück 10C ausgebildet, an das die Blutzuführ- und -rückführleitung 8, 9 angeschlossen sind (Fig. 1 ).

Die zweite Einrichtung 7 zum Fördern von Blut weist Mittel 11 zum Sammeln von Blut auf, die in der Blutrückführleitung 9 angeordnet sind. Die Mittel 11 zum Sammeln von Blut sind als ein Behälter mit einem vorgegebenen, abgeschlossenen Volumen ausgebildet. Nachfolgend werden die Mittel zum Sammeln von Blut als Blutsammelbehälter 11 bezeichnet.

Stromauf der Blutpumpe 6 ist an der Blutzuführleitung 7 ein arterielles Absperrorgan 12A zum Unterbrechen der Blutzuführleitung 8 und stromab des Blutsammelbehälters 11 ist an der Blutrückführleitung 9 ein venöses Absperrorgan 12B zum Unterbrechen der Blutrückführleitung angeordnet. Die Absperrorgane 12A, 12B können beispielsweise elektromagnetisch oder pneumatisch betätigbare Schlauchklemmen sein.

Der Blutsammelbehälter 11 weist einen Einlass 13 auf, zu dem ein erster Abschnitt 9A der Blutrückführleitung 9 führt, und weist einen Auslass 14 auf, von dem ein zweiter Abschnitt 9B der Blutrückführleitung 9 abgeht. Zur Detektion eines bestimmten Füllstandes im Blutsammelbehälter 11 ist ein Füllstandgeber 15 vorgesehen, der detektiert, wenn der Füllstand in dem Blutsammelbehälter einen vorgegebenen Wert erreicht. Darüber hinaus ist ein Druckgeber 16 vorgesehen, der den Druck im Blutsammelbehälter 11 misst.

Wenn der Blutsammelbehälter 11 mit Blut befüllt ist, verbleibt oberhalb des Flüssigkeitsspiegels 17 ein bestimmtes Luftvolumen in dem Blutsammelbehälter. Der Blutsammelbehälter 11 steht in Strömungsverbindung mit Mitteln 18 zum Speichern von Gas, insbesondere Luft, die als ein Behälter mit einem abgeschlossenen Volumen ausgebildet sind. Nachfolgend werden die Mittel 18 zum Speichern von Gas als Luftspeicher bezeichnet.

Damit Blutsammelbehälter 11 und Luftspeicher 18 miteinander kommunizieren können, geht von der Oberseite des Blutsammelbehälters eine Leitung 19 ab, die zu dem Luftspeicher 18 führt. In der Leitung 19 sind Mittel 20 zum Aufbau eines Drucks in dem Blutsammelbehälter 11 angeordnet, die beispielsweise als konventioneller Kompressor ausgebildet sein können. Solange der Kompressor 20 nicht betrieben wird, unterbricht der Kompressor die Strömungsverbindung zwischen Blutsammelbehälter 11 und Luftspeicher 18. Beim Betrieb des Kompressors 20 wird hingegen in dem Luftspeicher 18 befindliche Luft in den Blutsammelbehälter 11 überführt. Da die Luft komprimiert wird, baut sich in dem Blutsammelbehälter ein vorgegebener Druck auf. Der Druck wird mittels des geregelten Kompressors im Wesentlichen konstant gehalten.

Die Leitung 19 weist zwei Leitungsabschnitte 19A, 19B auf, von denen der eine Leitungsabschnitt 19A den Blutsammelbehälter 11 mit dem druckseitigen Anschluss 20A des Kompressors 20 und der andere Leitungsabschnitt 19B den saugseitigen Anschluss 20B des Kompressors 20 mit dem Luftspeicher 18 verbindet. Diese Leitungsabschnitte 19A, 19B bilden eine Verbindungsleitung 19 zum Überführen von Luft aus dem Luftspeicher in den Blutsammelbehälter.

Um bei nicht im Betrieb befindlichem Kompressor Luft aus dem Blutsammelbehälter in den Luftspeicher überführen zu können, ist eine Bypassleitung 21 vorgesehen, die von dem ersten Leitungsabschnitt 19A der Leitung 19 abgeht und zu dem zweiten Leitungsabschnitt 19B der Leitung 19 führt. In die Bypassleitung 21 ist ein Bypassventil 22 geschaltet. Zusammen mit den entsprechenden Leitungsabschnitten der Leitung 19 bildet die Bypassleitung 21 eine Verbindungsleitung zum Fördern von Luft aus dem Blutsammelbehälter in den Luftspeicher.

Um zu verhindern, dass Flüssigkeit aus dem Blutsammelbehälter 11 in den Luftspeicher 18 gelangen kann, ist in dem ersten Leitungsabschnitt 19A der Leitung 19 ein Filter 23 angeordnet, der eine hydrophobe, d.h. für Luft durchlässige, aber für Flüssigkeit undurchlässige Membran enthält.

Zum Be-/Entlüften des abgeschlossenen Volumens, das den Blutsammelbehälter und den Luftspeicher sowie die Leitung 19 umfasst, sind Mittel 24 zum Be-/Entlüften vorgesehen, die eine an den ersten Leitungsabschnitt 19A der Leitung 19 angeschlossene Be-/Entlüftungsleitung 24A mit einem Be-/Entlüftungsventil 24B aufweisen.

Neben dem Druckgeber 16 zum Messen des Drucks in dem Blutsammelbehälter 11 ist ein Druckgeber 25 zum Messen des Drucks in dem ersten Leitungsabschnitt 19A der Leitung 19 zwischen dem Filter 23 und dem Kompressor 20 und ein weiterer Druckgeber 26 zum Messen des Drucks im Luftspeicher 18 vorgesehen.

Die Blutbehandlungsvorrichtung kann noch über eine Substituatzufuhr für eine Prä- oder Postdilution aus einer nicht dargestellen Substituatquelle über eine nicht dargestellte Substituatleitung verfügen, die an die arteriellen Blutleitung 8 stromauf der Blutbehandlungseinheit 2 oder die venöse Blutleitung 9 stromab der Blutbehandlunsgeinheit 2 angeschlossen werden kann.

Die Blutbehandlungsvorrichtung verfügt über eine zentrale Steuereinheit 27, die über nicht dargestellte elektrische Leitungen mit der Blutpumpe 6, der arteriellen und venösen Schlauchklemme 12A, 12B, dem Bypassventil 22, dem Be-/Entlüftungsventil 24B, dem Füllstandsgeber 15, dem Kompressor 20 sowie den Druckgebern 16, 25 und 26 verbunden ist.

Nachfolgend wird der Betrieb der Dialysevorrichtung im Einzelnen beschrieben. Die zentrale Steuereinheit 27 steuert die einzelnen Komponenten der Blutbehandlungsvorrichtung wie folgt an.

Der Druck in dem Blutsammelbehälter 11 wird nachfolgend als Kammerdruck und der Druck in dem Luftspeicher 18 als Speicherdruck bezeichnet.

Zu Beginn der eigentlichen Dialysebehandlung wird eine Initialisierung des Systems durchgeführt, die in der WO 2008/148506 A2 beschrieben ist. Während des Betriebs der Blutbehandlungsvorrichtung schaltet die Steuereinheit fortlaufend zwischen einer arteriellen und venösen Phase um, wobei die Blutpumpe 6 sowohl in der arteriellen als auch der venösen Phase betrieben wird.

Zu Beginn der arteriellen Phase öffnet die Steuereinheit 27 das arterielle Absperrorgan 12A und schließt das venöse Absperrorgan 12B. Die Blutpumpe 6 wird in der arteriellen Phase mit einer vorgegebenen Förderrate betrieben, so dass in der Blutzuführleitung 8 Blut von dem Patientenanschluss 10 mit einer vorgegebenen Flussrate Q₍₁₎ₐ in den Blutsammelbehälter 11 gefördert wird. Das von der Blutpumpe 6 geförderte Volumen an Blut wird dem Patienten direkt entnommen. Beispielsweise fördert die Blutpumpe Blut mit einer Förderrate von 300 ml/min. Folglich füllt sich der Blutsammelbehälter 11 mit Blut. Dabei kann eine unerwünschte Rezirkulation nicht auftreten, da das venöse Absperrorgan 12B geschlossen ist. Der Kompressor 20 steht in der arteriellen Phase still.

Das Bypassventil 22 wird von der Steuereinheit 27 in der arteriellen Phase geöffnet, so dass die aus dem Blutsammelbehälter 11 verdrängte Luft über die Bypassleitung 21 in den Luftspeicher 18 gelangt. Folglich steigt der Speicherdruck an. Bei dem Bypassventil handelt es sich besonders bevorzugt um ein digitales Ventil, das im geöffneten Zustand ungeregelt ist. Ein solches digitales Ventil ist besonders kostengünstig. Das digitale Ventil wird getaktet betrieben, um die Druckschwankungen klein zu halten. Die Taktung der Öffnungszeiten des Ventils kann besonders bevorzugt mit einer Frequenz von 1 Hz bis 6 Hz erfolgen. Alternativ ist auch die Verwendung eines proportionalen Ventils möglich, was aber mit höheren Kosten verbunden ist.

Sobald die in dem Blutsammelbehälter 11 und dem Leitungsvolumen enthaltene Luftmasse einen vorgegebenen Betrag erreicht hat, der sich aus dem gewünschten Schlagvolumen und dem gewünschten Rückgabedruck ergibt, schließt die Steuereinheit 27 das Bypassventil 22. Folglich entstehen zwei getrennte Luftvolumina, d.h. das Luftvolumen in dem Blutsammelbehälter mit den zugehörigen Leitungsabschnitten und das Volumen des Luftspeichers mit den zugehörigen Leitungsabschnitten.

In der venösen Phase ist das arterielle Absperrorgan 12A geöffnet und das Bypassventil 22 geschlossen. Die Steuereinheit 27 öffnet in der venösen Phase das venöse Absperrorgan 12B. Die Blutpumpe 6 wird in der venösen Phase mit einer Förderrate Q₍₁₎ᵥ betrieben, die gleich der Förderrate der Blutpumpe in der arteriellen Phase Q₍₁₎ᵥ = Q₍₁₎ₐ, beispielsweise 300 ml/min, sein kann. Die Förderrate der Blutpumpe 6 in der venösen Phase kann aber auch größer oder kleiner als die Förderrate in der arteriellen Phase sein.

Der Kompressor 20 wird in der venösen Phase betrieben und fördert Luft aus dem Luftspeicher 18 in den Blutsammelbehälter 11, um einen Überdruck aufzubauen, so dass Blut aus dem Blutsammelbehälter gefördert wird. Der Kompressor 20 wird dabei derart betrieben, dass sich in dem Blutsammelbehälter 11 der gewünschte Rückgabedruck einstellt. Mit der Vorgabe eines bestimmten Rückgabedrucks stellt sich ein korrespondierender Blutfluss ein. Der Rückgabedruck wird dabei so vorgegeben, dass der sich einstellende Blutfluss dem Sollwert entspricht. Es erfolgt also eine Regelung des Rückgabedrucks, um den korrespondierenden Blutfluss auf den Sollwert einzustellen. Da dem Blutsammelbehälter 11 fortwährend Luft aus dem Luftspeicher 18 zugeführt wird, nimmt der Speicherdruck kontinuierlich ab.

Aufgrund des gleichzeitigen Betriebs der Blutpumpe 6 und des Kompressors 20 in der venösen Phase stellt sich in der Blutrückführleitung 9 stromab des Blutsammelbehälters 11 ein Blutfluss ein, der sich aus der Summe des von der Blutpumpe 6 geförderten Volumens an Blut Q₍₁₎ᵥ und des aus dem Blutsammelbehälter 11 verdrängten Volumens an Blut Q₍₂₎ᵥ ergibt. Da die Blutpumpe 6 auch während der venösen Phase betrieben wird, teilen sich die Flüsse Q₍₁₎ᵥ und Q₍₂₎ᵥ an dem Patientenanschluss 10 wieder auf. Dabei bestimmt allein die Geschwindigkeit, mit der der Pegel in dem Blutsammelbehälter 11 abgesenkt wird, den venösen Rückgabefluss Q₍₂₎ᵥ in den Patienten. Dieser Rückgabefluss Q₍₂₎ᵥ ist von dem Zirkulationsfluss Q₍₁₎ᵥ unabhängig, der sich in dem geschlossenen Kreislauf einstellt, der die arterielle und venöse Blutleitung 8, 9 und die Blutkammer 4 des Dialysators 2 umfasst. Aufgrund des kontinuierlichen Betriebs der Blutpumpe 6 in der venösen und arteriellen Phase wird die Blutkammer 4 des Dialysators 2 kontinuierlich von Blut durchströmt. Die Dialysierflüssigkeitskammer 5 wird stets kontinuierlich von Dialysierflüssigkeit durchströmt.

Bei dem vorliegenden Ausführungsbeispiel wird der Kompressor so betrieben, dass sich der für die Einstellung des gewünschten Blutflusses erforderliche Rückgabedruck aufbaut. Dabei wird der Kompressor so geregelt, dass der Rückgabedruck im Wesentlichen konstant gehalten wird, so dass sich auch ein im Wesentlichen konstanter Blutfluss ergibt.

Eine alternative Ausführungsform sieht nicht die Regelung des Rückgabedrucks, sondern die Regelung des Volumenstroms in dem Blutsammelbehälter vor, so dass sich der korrespondierende Rückgabedruck in dem Blutsammelbehälter einstellt. Bei der alternativen Ausführungsform wird analog einer Füllstandsmessung mit dem Drucksensor 16 der Druck zu zwei Zeitpunkten in dem Blutsammelbehälter 11 gemessen. Die Messung des Drucks zu jeweils zwei Zeitpunkten kann fortlaufend über die gesamte Behandlungszeit erfolgen. Aus den beiden Druckmesswerten für die beiden Zeitpunkte wird die Änderung des Volumens an Blut in dem Blutsammelbehälter 11 und aus der Volumenänderung pro Zeiteinheit der Volumenstrom berechnet. Diese Berechnung kann während der Behandlung fortlaufend erfolgen. Die Drehzahl des Kompressors 200 wird so geregelt, dass der berechnete Volumenstrom dem vorgegebenen Sollfluss entspricht. Der Rückgabedruck stellt sich bei dieser Ausführungsform also als Folge der Kompressordrehzahl ein.

## Patentansprüche

1. Extrakorporale Blutbehandlungsvorrichtung für den Betrieb mit einem einzigen Patientenanschluss, der mit einer arteriellen und einer venösen Blutleitung eines extrakorporalen Blutkreislaufs verbunden ist,
wobei die Blutbehandlungsvorrichtung aufweist:
eine erste Einrichtung (6) zum Fördern von Blut,
eine zweite Einrichtung (7) zum Fördern von Blut, die Mittel (11) zum Sammeln von Blut, das in der venösen Blutleitung (8) aus einer Blutbehandlungseinheit (2) strömt, und Mittel (20) zum Aufbau eines Überdrucks in den Mitteln zum Sammeln von Blut aufweist, so dass in den Mitteln zum Sammeln von Blut gesammeltes Blut zu dem Patientenanschluss (10) strömt,
ein arterielles Absperrorgan (12A) zum Unterbrechen der Flüssigkeitsströmung in der arteriellen Blutleitung (8) und ein venöses Absperrorgan (12B) zum Unterbrechen der Flüssigkeitsströmung in der venösen Blutleitung (9),
eine Steuereinheit (27) zum Ansteuern der ersten und zweiten Einrichtung (6, 7) zum Fördern von Blut sowie zum Ansteuern des arteriellen und venösen Absperrorgans (12A, 12B), wobei die Steuereinheit (27) derart konfiguriert ist, dass die erste Einrichtung (6) zum Fördern von Blut und die Mittel (20) zum Aufbau eines Überdrucks in den Mitteln (11) zum Sammeln von Blut sowie das arterielle und venöse Absperrorgan (12A, 12B) derart angesteuert werden, dass
in einer arteriellen Phase die erste Einrichtung (6) zum Fördern von Blut bei geöffnetem arteriellen Absperrorgan (12A) und geschlossenem venösen Absperrorgan (12B) mit einer vorgegebenen ersten Förderrate betrieben wird, so dass Blut mit einer vorgegebenen ersten Flussrate (Q₍₁₎ₐ) von dem Patientenanschluss (10) über die Blutbehandlungseinheit (2) in die Mittel (11) zum Sammeln von Blut strömt, und
**dadurch gekennzeichnet, dass** die Steuereinheit (27) weiter derart konfiguriert ist, dass in einer sich an die arterielle Phase anschließenden venösen Phase die erste Einrichtung (6) zum Fördern von Blut bei geöffnetem arteriellen und venösen Absperrorgan (12A, 12B) mit einer vorgegebenen zweiten Förderrate betrieben wird, so dass Blut mit einer vorgegebenen zweiten Flussrate (Q₍₁₎ᵥ) von dem Patientenanschluss (10) über die Blutbehandlungseinheit (2) in die Mittel (11) zum Sammeln von Blut strömt, und
in der venösen Phase mit den Mitteln (20) zum Aufbau eines Überdrucks in den Mitteln (11) zum Sammeln von Blut ein Überdruck aufgebaut wird, so dass Blut mit einer vorgegebenen dritten Flussrate aus den Mitteln (11) zum Sammeln von Blut zu dem Patientenanschluss (10) strömt,
wobei fortlaufend zwischen der arteriellen und venösen Phase umgeschaltet wird.

2. Blutbehandlungsvorrichtung nach Anspruch 1, **dadurch gekennzeichnet, dass** die erste Flussrate (Q₍₁₎ₐ) gleich der zweiten Flussrate (Q₍₁₎ᵥ) ist.

3. Blütbehandlungsvorrichtung nach Anspruch 1, **dadurch gekennzeichnet, dass** die erste Flussrate (Q₍₁₎ₐ) größer oder kleiner als die zweite Flussrate (Q₍₁₎ᵥ) ist.

4. Blutbehandlungsvorrichtung nach einem der Ansprüche 1 bis 3, **dadurch gekennzeichnet, dass** der einzige Patientenanschluss als eine Kanüle (10) mit einem distalen und proximalen Ende (10A, 10B) ausgebildet ist, wobei an dem proximalen Ende (10B) der Kanüle (10) ein die arterielle und venöse Blutleitung (8, 9) verbindendes Verbindungsstück (10C) vorgesehen ist.

5. Blutbehandlungsvorrichtung nach einem der Ansprüche 1 bis 4, **dadurch gekennzeichnet, dass** die erste Einrichtung zum Fördern von Blut als eine okkludierende Blutpumpe (6) ausgebildet ist.

6. Blutbehandlungsvorrichtung nach einem der Ansprüche 1 bis 5, dadurch gekenntzeichnet, dass die zweite Einrichtung (7) zum Fördern von Blut Mittel (18) zum Speichern von Luft aufweist, die ein abgeschlossenes Volumen umfassen, wobei die Mittel (20) zum Aufbau eines Überdrucks derart mit den Mitteln (18) zum Speichern von Luft und den Mitteln (11) zum Sammeln von Blut in Strömungsverbindung stehen, dass Luft aus den Mitteln (18) zum Speichern von Luft unter Verdrängung des in den Mitteln (11) zum Sammeln von Blut gesammelten Bluts in die Mittel (11) zum Sammeln von Blut überführbar ist.

7. Blutbehandlungsvorrichtung nach Anspruch 6, **dadurch gekennzeichnet, dass** die zweite Einrichtung (7) zum Fördern von Blut eine die Mittel (18) zum Speichern von Luft und die Mittel (11) zum Sammeln von Blut verbindende Bypassleitung (21) aufweist, wobei in der Bypassleitung (21) ein Bypassventil (22) angeordnet ist.

## Claims

1. Extracorporeal blood treatment device for operation with a single patient connection, which is connected to an arterial and a venous blood line of an extracorporeal blood circuit,
wherein the blood treatment device comprises:
a first apparatus (6) for conveying blood,
a second apparatus (7) for conveying blood, which comprises means (11) for collecting blood that flows in the venous blood line (8) from a blood treatment unit (2) and means (20) for establishing an overpressure in the means for collecting blood, so that blood collected in the means for collecting blood flows to the patient connection (10),
an arterial closure element (12A) or interrupting the flow of liquid in the arterial blood line (8) and a venous closure element (12B) or interrupting the flow of liquid in the venous blood line (9),
a control unit (27) for actuating the first and second apparatuses (6, 7) for conveying blood, as well as actuating the arterial and venous closure elements (12A, 12B),
wherein
the control unit (27) is configured so that the first apparatus (6) for conveying blood and the means (20) for establishing an overpressure in the means (11) for collecting blood as well as the arterial and venous closure element (12A, 12B) are actuated so that
in an arterial phase, the first apparatus (6) for conveying blood is operated with open arterial closure element (12A) and closed venous closure element (12B) at a predetermined first delivery rate, so that blood flows at a predetermined first flow rate (Q₍₁₎ₐ) from the patient connection (10) via the blood treatment unit (2) into the means (11) for collecting blood, and
**characterised in that** the control unit (27) is further configured so that in a venous phase following the arterial phase, the first apparatus (6) for conveying blood is operated with open arterial and closed venous closure element (12A, 12B) at a predetermined second delivery rate, so that blood flows at a predetermined second flow rate (Q₍₁₎ᵥ) from the patient connection (10) via the blood treatment unit (2) into the means (11) for collecting blood, and
in the venous phase, overpressure is established in the means (11) for collecting blood with the means (20) for establishing overpressure, so that blood flows at a predetermined third flow rate from the means (11) for collecting blood to the patient connection (10),
wherein there is continuous switching between the arterial and venous phases.

2. Blood treatment device in accordance with claim 1, **characterised in that** the first flow rate (Q₍₁₎ₐ) is the same as the second flow rate (Q₍₁₎ᵥ).

3. Blood treatment device in accordance with claim 1, **characterised in that** the first flow rate (Q₍₁₎ₐ) is greater or smaller than the second flow rate (Q₍₁₎ᵥ).

4. Blood treatment device in accordance with one of the claims 1 to 3, **characterised in that** the single patient connection is configured as a cannula (10) with a distal and a proximal end (10A, 10B), wherein a connection piece (10C) connecting the arterial and venous blood lines (8, 9) is provided at the proximal end (10B) of the cannula.

5. Blood treatment device in accordance with one of the claims 1 to 4, **characterised in that** the first apparatus for conveying blood is configured as an occluding blood pump (6).

6. Blood treatment device in accordance with one of the claims 1 to 5, **characterised in that** the second apparatus (7) for conveying blood comprises means (18) for storing air, which comprises a closed volume, wherein the means (20) for establishing an overpressure are in flow connection with the means (18) for storing air and the means (11) for collecting blood, so that air can be transferred from the means (18) for storing air while displacing the blood collected in the means (11) for collecting blood into the means (11) for collecting blood.

7. Blood treatment device in accordance with claim 6, **characterised in that** the second apparatus (7) for conveying blood has a bypass line (21) connecting the means (18) for storing air and the means (11) for collecting blood, wherein a bypass valve (22) is positioned in the bypass line (21).

## Revendications

1. Dispositif extracorporel de traitement du sang pour le fonctionnement avec un seul raccordement au patient, qui est relié à un conduit sanguin artériel et un conduit sanguin veineux d'une circulation sanguine extracorporelle,
dans lequel le dispositif de traitement du sang présente :
un premier système (6) servant au transport du sang,
un deuxième système (7) servant au transport du sang, qui comprend des moyens (11) servant à la collecte du sang qui circule dans le conduit sanguin veineux (9) d'une unité de traitement du sang (2), et des moyens (20) servant à la constitution d'une surpression dans les moyens servant à la collecte du sang si bien que le sang collecté dans les moyens servant à la collecte du sang circule jusqu'au raccordement au patient (10),
un organe d'obturation artérielle (12A) servant à l'interruption de la circulation du fluide dans le conduit sanguin artériel (8) et un organe d'obturation veineuse (12B) servant à l'interruption de la circulation du fluide dans le conduit sanguin veineux (9),
une unité de commande (27) servant à commander le premier et le deuxième système (6, 7) servant au transport du sang et à commander l'organe d'obturation artérielle et veineuse (12A, 12B),
dans lequel l'unité de commande (27) est configurée de telle sorte que le premier système (6) servant au transport du sang et les moyens (20) servant à constituer une surpression dans les moyens (11) servant à la collecte du sang ainsi que l'organe d'obturation artérielle et veineuse (12A, 12B) soient commandés de telle sorte que, au cours d'une phase artérielle, le premier système (6) servant au transport du sang fonctionne à un premier taux de transport prédéfini lorsque l'organe d'obturation artérielle (12A) est ouvert et l'organe d'obturation veineuse (12B) est fermé si bien que le sang circule à un premier débit prédéfini (Q_{(I)a}) depuis le raccordement au patient (10) jusqu'aux moyens (11) servant à la collecte du sang en passant par l'unité de traitement du sang (2), et
**caractérisé en ce que** l'unité de commande (27) est en outre configurée de telle sorte que, au cours d'une phase veineuse s'adjoignant à la phase artérielle, le premier système (6) servant au transport du sang fonctionne à un deuxième taux de transport prédéfini lorsque l'organe d'obturation artérielle et veineuse (12A, 12B) est ouvert si bien que le sang circule à un deuxième débit prédéfini (Q_{(I)v}) depuis le raccordement au patient (10) jusqu'aux moyens (11) servant à la collecte du sang en passant par l'unité de traitement du sang (2), et
au cours de la phase veineuse, une surpression est constituée avec les moyens (20) servant à constituer une surpression dans les moyens (11) servant à la collecte du sang si bien que le sang circule à un troisième débit prédéfini depuis les moyens (11) servant à la collecte du sang jusqu'au raccordement au patient (10),
la commutation entre la phase artérielle et la phase veineuse s'effectuant en continu.

2. Dispositif extracorporel de traitement du sang selon la revendication 1, **caractérisé en ce que** le premier débit (Q_{(I)a}) est identique au deuxième débit (Q_{(I)v}).

3. Dispositif extracorporel de traitement du sang selon la revendication 1, **caractérisé en ce que** le premier débit (Q_{(I)a}) est supérieur ou inférieur au deuxième débit (Q_{(I)v}).

4. Dispositif extracorporel de traitement du sang selon l'une des revendications 1 à 3, **caractérisé en ce que** le seul raccordement au patient est conçu sous la forme d'une canule (10) pourvue d'une extrémité distale et d'une extrémité proximale (10A, 10B), une pièce de liaison (10C) reliant le conduit sanguin artériel et veineux (8, 9) étant prévue à l'extrémité proximale (10B) de la canule (10).

5. Dispositif extracorporel de traitement du sang selon l'une des revendications 1 à 4, **caractérisé en ce que** le premier système servant au transport du sang est conçu sous la forme d'une pompe sanguine occlusive (6).

6. Dispositif extracorporel de traitement du sang selon l'une des revendications 1 à 5, **caractérisé en ce que** le deuxième système (7) servant au transport du sang comprend des moyens (18) servant au stockage de l'air, qui comprennent un volume clos, dans lequel les moyens (20) servant à constituer une surpression sont en communication de fluide avec les moyens (18) servant au stockage de l'air et les moyens (11) servant à la collecte du sang de telle sorte que l'air sortant des moyens (18) servant au stockage de l'air peut être transféré jusque dans les moyens (11) servant à la collecte du sang en refoulant le sang collecté dans les moyens (11) servant à la collecte du sang.

7. Dispositif extracorporel de traitement du sang selon la revendication 6, **caractérisé en ce que** le deuxième système (7) servant au transport du sang comprend un conduit de dérivation (21) reliant les moyens (18) servant au stockage de l'air et les moyens (11) servant à la collecte du sang, une vanne de dérivation (22) étant disposée dans le conduit de dérivation (21).
